# DEMANDE DE BREVET EUROPEEN

(11) **EP 0 976 419 A1**
(43) Date de publication de la demande: **02.02.2000**
(21) Numéro de dépôt: 99420165.5
(22) Date de dépôt: 16.07.1999
(51) Int. Cl.: A61M 39/26

(54) **Dispositif de connexion pour ligne de dialyse péritonéale**

(30) Priorité: 29.07.1998 FR 9809943
(71) Demandeur: Laboratoire Aguettant, 69007 Lyon (FR)
(72) Inventeur: Frezza, Pierre, 69390 Charly (FR)
(74) Mandataire: Maureau, Philippe

(57) **Abrégé**

Dispositif comprenant un connecteur (2) monté à une extrémité d'une ligne de dialyse et pouvant être relié à un connecteur (4) associé à un récipient (2) caractérisé en ce que le connecteur (2) monté sur la tubulure débouchant dans la cavité péritonéale est équipé d'une pince (19, 20) soumise à l'action d'un ressort (24) agissant dans un sens de fermeture de celle-ci, cette pince étant équipée de moyens d'actionnement (22) assurant son ouverture lorsque le connecteur (2) est accouplé à un connecteur complémentaire (4) et permettant sa fermeture par action du ressort (24) lorsque le connecteur est désaccouplé.

## Description

La présente invention a pour objet un dispositif de connexion pour ligne de dialyse péritonéale. La dialyse péritonéale est une alternative à l'hémodialyse dans le traitement de l'insuffisance rénale. La dialyse péritonéale utilise la cavité péritonéale comme zone d'échange.

La technique consiste à introduire puis à retirer du péritoine un liquide à intervalles réguliers. Cette technique nécessite l'implantation chirurgicale d'un drain dans la cavité péritonéale du patient, ce drain débouchant à l'extérieur du corps par une tubulure souple terminée par un connecteur. Ce connecteur permet de relier la tubulure soit à un récipient contenant un soluté destiné à être introduit dans le péritoine, soit à un récipient de drainage destiné à récupérer le liquide contenu dans le péritoine.

La cavité péritonéale doit toujours être bactériologiquement isolée du milieu extérieur, pour éviter les risques d'infection. A cet effet, les dispositifs de connexion existants comportent un dispositif d'obturation de la ligne par clampage de la tubulure souple, ce dispositif agissant soit sur la tubulure souple, soit au niveau du connecteur. Le plus souvent, l'ouverture ou la fermeture du circuit doit être faite volontairement. Il en résulte des risques septiques, dans la mesure où la tubulure peut rester en position ouverte, en cas d'oubli d'une manoeuvre.

Le document EP 0 263 789 concerne un dispositif de connexion comportant deux mâchoires soumises à l'action d'éléments élastiques, qui provoquent le serrage des mâchoires et l'obturation d'une tubulure souple lorsque les deux parties du connecteur sont désassemblées, la connexion permettant d'agir sur les mâchoires dans un sens d'écartement l'une de l'autre pour permettre le passage de fluide dans la tubulure souple.

Le document DE 91 05 229 concerne un dispositif de connexion permettant une fermeture automatique d'un conduit lorsque les deux pièces du connecteur sont désolidarisées l'une de l'autre. Dans ce cas, il ne s'agit pas de réaliser la fermeture du passage par pincement d'une tubulure souple, mais par déplacement axial d'une partie en forme de clapet, qui est libérée de son siège à l'encontre de l'action d'un ressort.

Le but de l'invention est de fournir un dispositif de connexion pour ligne de dialyse péritonéale, qui ouvre et ferme automatiquement le circuit, respectivement dès que l'on connecte et déconnecte le connecteur, et qui soit réalisé avec un nombre réduit de pièces simples. Il en résulte une simplification des manoeuvres, en évitant de plus tout risque d'oubli de fermeture de l'accès au péritoine avant déconnexion volontaire ou accidentelle.

A cet effet, le dispositif de connexion qu'elle concerne, du type comportant une tubulure souple dont une extrémité débouche dans la cavité péritonéale d'un patient et dont l'autre extrémité, débouchant à l'extérieur du corps du patient, est équipée d'un connecteur pouvant être accouplé à un connecteur complémentaire monté sur un récipient contenant un soluté, ou un récipient de drainage, et dont le connecteur monté sur la tubulure débouchant dans la cavité péritonéale est équipé d'une pince équipée de moyens d'actionnement assurant son ouverture lorsque le connecteur est accouplé à un connecteur complémentaire et permettant sa fermeture lorsque le connecteur est désaccouplé, est caractérisé en ce que le connecteur monté en bout de la tubulure débouchant dans la cavité péritonéale comprend un embout comportant d'une part une zone d'accouplement étanche avec l'autre connecteur et d'autre part une partie tubulaire sur laquelle est emmanchée la tubulure souple, cet embout étant équipé d'une pince comportant deux mors susceptibles de pincer la tubulure pour l'obturer, l'embout étant monté coulissant à l'encontre de l'action d'un ressort, dans un corps tubulaire équipé de moyens de liaison par vissage à l'autre connecteur, entre une position sortie correspondant à la position de fermeture de la pince, lorsque le connecteur est désaccouplé et une position rentrée correspondant à la position d'ouverture de la pince, lorsque le connecteur est accouplé.

Lorsque le connecteur monté en bout de la tubulure souple est désaccouplé, la pince dont il est équipé ferme la tubulure souple. Lorsqu'un accouplement est réalisé avec un connecteur relié à une poche de soluté ou à une poche de drainage, l'embout prenant appui dans ce second connecteur coulisse vis à vis du connecteur dans lequel il est monté, assurant l'ouverture de la pince et par suite l'ouverture de la tubulure souple.

Avantageusement, ce dispositif comporte un manchon engagé sur l'embout et bloqué axialement sur celui-ci, prolongé du côté de la tubulure et au-delà d'un épaulement par deux pattes symétriques en bout desquelles sont disposés deux mors en vis-à-vis, ce manchon étant monté coulissant dans le corps tubulaire, qui comporte une collerette présentant une ouverture centrale pour le passage des pattes de la pince, qui délimitent une surface extérieure conique dont la plus grande section est située du côté de l'extrémité libre des pattes, la distance radiale entre les deux extrémités de la surface conique de chaque mors étant au moins égale au rayon de la tubulure souple, un ressort hélicoïdal entourant les pattes et prenant appui d'une part sur l'épaulement du manchon et d'autre part sur la collerette du corps tubulaire.

Suivant d'autres caractéristiques de l'invention l'extrémité du manchon engagée sur l'embout comporte un bossage annulaire intérieur destiné à être engagé dans une gorge annulaire extérieure que comporte l'embout. La surface conique délimitée par les pattes est limitée du côté de sa plus grande section par un épaulement formant une butée d'appui contre la collerette du corps tubulaire, en position de fermeture de la pince.

En outre, suivant une forme d'exécution, le connecteur monté en bout de la tubulure souple présente un filetage extérieur et est un connecteur mâle, tandis que l'autre connecteur est un connecteur femelle et comporte un taraudage. L'ouverture et la fermeture de la pince sont donc réalisées respectivement par vissage du connecteur mâle dans le connecteur femelle, et dévissage du connecteur mâle vis à vis du connecteur femelle.

De toute façon l'invention sera bien comprise à l'aide de la description qui suit, en référence au dessin schématique annexé, représentant, à titre d'exemple non limitatif, une forme d'exécution de ce dispositif de connexion.
- Figure 1 est une vue en perspective éclatée des différentes pièces constitutives de ce dispositif,
- Figures 2 et 3 sont deux vues en coupe longitudinale et à échelle agrandie de ce dispositif, respectivement en position désaccouplée et en position accouplée des deux connecteurs.

Le dispositif représenté au dessin comprend un premier connecteur 2 monté à une extrémité d'une tubulure souple 3 dont l'autre extrémité débouche à l'intérieur du péritoine du patient, et un second connecteur 4 relié à une poche ou autre récipient contenant un soluté, ou un récipient de drainage, par une tubulure 5. De façon connue en soi, le connecteur 4 est un connecteur femelle, présentant une partie cylindrique 6 prolongeant une partie tubulaire 7 de plus petite section, sur laquelle est engagée la tubulure 5. La partie cylindrique 6 comporte un taraudage 8.

Le connecteur 2 comprend un corps tubulaire 9 dont l'extrémité avant est ouverte, et dont l'extrémité arrière située du côté de la tubulure souple 9 est obturée par un capot 10 fixé par encliquetage et présentant une ouverture centrale pour le passage de la tubulure. Le corps tubulaire comporte un filetage extérieur 11 complémentaire du taraudage 8 du connecteur 4.

Le corps tubulaire 9 est équipé à proximité de son extrémité postérieure d'une collerette annulaire intérieure 12 délimitant une ouverture centrale.

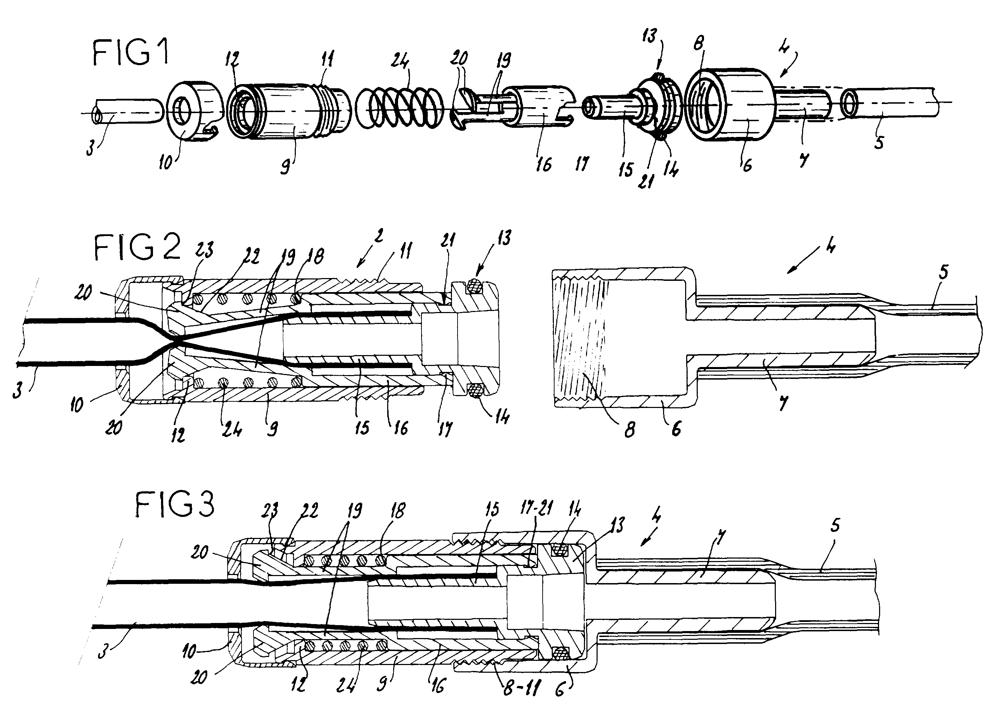

Le connecteur 2 comprend également un embout 13 dont l'extrémité avant destinée à être introduite dans le connecteur 4 est équipée d'un joint torique 14 destiné à assurer l'étanchéité. Cet embout 13 se poursuit vers l'arrière par une partie tubulaire 15 de plus faible section sur laquelle est emmanché le tube souple 3. Sur la zone avant de l'embout est engagé un manchon 16 dont l'extrémité avant possède un bossage annulaire intérieur 17 engagé dans une gorge annulaire extérieure 21 de l'embout 13 pour réaliser un blocage axial de ces deux pièces l'une par rapport à l'autre. Le manchon 16 est monté coulissant à l'intérieur du corps tubulaire 9. L'extrémité postérieure du manchon 16 présente un épaulement 18, à partir duquel s'étendent deux pattes 19 diamétralement opposées, se terminant par deux mors 20. Chaque mors 20 présente, sur sa face extérieure, une surface tronconique 22 dont la plus grande section est située du côté de l'extrémité postérieure. Chaque surface tronconique est limitée à son extrémité postérieure par un épaulement 23. La surface tronconique 22 constituée par les surfaces extérieures des deux mors 20 est montée, au niveau de l'ouverture délimitée par la collerette 12 du corps tubulaire, à l'intérieur même de cette ouverture. Le diamètre extérieur au niveau des mors est tel que, lorsque les pattes 19 sont écartées, comme montré à la figure 3, la surface conique puisse être logée à l'intérieur du capot 10 du corps tubulaire. La surface conique 22 forme un cône de serrage à l'intérieur de la collerette 12, le débattement de chaque patte étant sensiblement égal au rayon de la tubulure souple 3. Il est prévu enfin, comme montré au dessin, un ressort 24 prenant appui d'une part sur la collerette 12 et d'autre part sur l'épaulement 18 du manchon 16.

En l'absence d'accouplement entre les deux connecteurs 2 et 4, comme montré à la figure 2, le ressort 24 tend à pousser le manchon 16 et l'embout 13 vers l'extérieur, le cône de serrage constitué par la surface 22 rapprochant les deux mors 20 de la pince et écrasant la tubulure souple 3 dans un sens de fermeture de celle-ci.

Lorsque l'on accouple les connecteurs 2 et 4, l'embout 13 vient dans le fond de la partie tubulaire 6 du connecteur 4, où il est bloqué en translation. Le vissage du connecteur 2 dans le connecteur 4 se traduit par un déplacement relatif de l'embout 13 et du manchon 16 vis à vis du corps tubulaire dans un sens de pénétration dans ce dernier. Le ressort 24 se comprime, et le cône de serrage 22 prend appui dans l'ouverture de la collerette 12, par une zone de plus faible section, assurant l'écartement des mors 20 et par suite l'ouverture de la tubulure souple comme montré à la figure 3.

Lorsque l'on désolidarise les connecteurs 2 et 4, l'embout 13 et le manchon 16 sont déplacés par le ressort 24 dans un sens de sortie vis à vis du corps tubulaire 9, ce mouvement étant accompagné par un rapprochement des mors 20 de la pince et une fermeture de la tubulure 3, comme montré à la figure 2.

Comme il ressort de ce qui précède l'invention apporte une grande amélioration à la technique existante en fournissant un dispositif permettant de réaliser automatiquement la fermeture d'une tubulure de dialyse péritonéale lors de la déconnexion de deux connecteurs, et l'ouverture de cette tubulure souple lors de l'assemblage de ces deux connecteurs.

Comme il va de soi l'invention ne se limite pas à la seule forme d'exécution de ce dispositif, décrite ci-dessus à titre d'exemple, elle en embrasse au contraire toutes les variantes. C'est ainsi notamment que la pince de fermeture de la tubulure souple pourrait être agencée différemment et notamment que les mors de cette pince pourraient être articulés à l'intérieur du corps tubulaire et poussés par le manchon, ou encore que les mors de la pince pourraient être disposés à l'extérieur du connecteur 2, et associés chacun à un levier actionné par l'extrémité avant du connecteur 4, sans que l'on sorte pour autant du cadre de l'invention.

## Revendications

1. Dispositif de connexion pour ligne de dialyse péritonéale, du type comportant une tubulure souple (3) dont une extrémité débouche dans la cavité péritonéale d'un patient et dont l'autre extrémité, débouchant à l'extérieur du corps du patient, est équipée d'un connecteur (2) pouvant être accouplé à un connecteur (4) complémentaire monté sur un récipient contenant un soluté, ou un récipient de drainage, et dont le connecteur (2) monté sur la tubulure débouchant dans la cavité péritonéale est équipé d'une pince (19, 20) équipée de moyens d'actionnement (22) assurant son ouverture lorsque le connecteur (2) est accouplé à un connecteur complémentaire (4) et permettant sa fermeture lorsque le connecteur est désaccouplé, caractérisé en ce que le connecteur (2) monté en bout de la tubulure (3) débouchant dans la cavité péritonéale comprend un embout (13) comportant d'une part une zone d'accouplement étanche avec l'autre connecteur et d'autre part une partie tubulaire (15) sur laquelle est emmanchée la tubulure souple (3), cet embout (13) étant équipé d'une pince (19) comportant deux mors (20) susceptibles de pincer la tubulure pour l'obturer, l'embout (13) étant monté coulissant à l'encontre de l'action d'un ressort (24), dans un corps tubulaire (9) équipé de moyens de liaison par vissage à l'autre connecteur (4), entre une position sortie correspondant à la position de fermeture de la pince, lorsque le connecteur (2) est désaccouplé et une position rentrée correspondant à la position d'ouverture de la pince, lorsque le connecteur (2) est accouplé.

2. Dispositif selon la revendication 1, caractérisé en ce qu'il comporte un manchon (16) engagé sur l'embout (13) et bloqué axialement sur celui-ci, prolongé du côté de la tubulure (3) et au-delà d'un épaulement (18) par deux pattes symétriques (19) en bout desquelles sont disposés deux mors (20) en vis-à-vis, ce manchon (16) étant monté coulissant dans le corps tubulaire (9), qui comporte une collerette (12) présentant une ouverture centrale pour le passage des pattes (19) de la pince qui délimitent une surface extérieure conique (22) dont la plus grande section est située du côté de l'extrémité libre des pattes, la distance radiale entre les deux extrémités de la surface conique de chaque mors étant au moins égale au rayon de la tubulure souple, un ressort hélicoïdal (24) entourant les pattes et prenant appui d'une part sur l'épaulement (18) du manchon (16) et d'autre part sur la collerette (12) du corps tubulaire.

3. Dispositif selon la revendication 2, caractérisé en ce que l'extrémité du manchon (16) engagée sur l'embout (13) comporte un bossage annulaire intérieur (17) destiné à être engagé dans une gorge annulaire extérieure (21) que comporte l'embout.

4. Dispositif selon l'une quelconque des revendications 2 et 3, caractérisé en ce que la surface conique (22) délimitée par les pattes est limitée du côté de sa plus grande section par un épaulement (23) formant une butée d'appui contre la collerette (12) du corps tubulaire, en position de fermeture de la pince.

5. Dispositif selon l'une quelconque des revendications 2 à 4, caractérisé en ce que le connecteur (2) monté en bout de la tubulure souple présente un filetage extérieur (11) et est un connecteur mâle, tandis que l'autre connecteur (4) est un connecteur femelle et comporte un taraudage (8).
